(19) Europäisches Patentamt
European Patent Office
Office européen des brevets

(11) Publication number: **0 017 679 B1**

# (12) EUROPEAN PATENT SPECIFICATION

(45) Date of publication of patent specification: **24.02.82**

(21) Application number: **79300599.2**

(22) Date of filing: **11.04.79**

(51) Int. Cl.[3]: **C 07 D 401/14,** C 07 D 401/06, A 61 K 31/505, C 07 D 417/14

## (54) Pyridylalkylpyrimidone compounds, process for preparing them and pharmaceutical compositions containing them.

(43) Date of publication of application: **29.10.80 Bulletin 80/22**

(45) Publication of the grant of the patent: **24.02.82 Bulletin 82/8**

(84) Designated Contracting States: **BE CH DE FR GB IT LU NL SE**

(56) References cited:
**GB - A - 1 419 994**
**US - A - 4 145 546**

(73) Proprietor: **SMITH KLINE & FRENCH LABORATORIES LIMITED**
**Mundells**
**Welwyn Garden City Hertfordshire, AL7 1EY (GB)**

(72) Inventor: **Brown, Thomas Henry**
**115 Daniells**
**Welwyn Garden City Hertfordshire (GB)**
Inventor: **Durant, Graham John**
**401 Knightsfield**
**Welwyn Garden City Hertfordshire (GB)**
Inventor: **Ganellin, Charon Robin**
**"Kinwood" Briary Wood End**
**Welwyn Hertfordshire (GB)**

(74) Representative: **Waters, David Martin, Dr. et al,**
**P.O. Box 39**
**Welwyn Garden City, Hertfordshire AL7 1EY (GB)**

**EP 0 017 679 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

Pyridylalkylpyrimidone compounds, process for preparing them and pharmaceutical compositions containing them.

This invention relates to pharmacologically active pyridylalkylpyrimidone compounds, to a process for preparing them and to pharmaceutical compositions containing them.

In German Offenlegungsschrift 2658267 a group of pyrimidones (1) are described as having histamine $H_1$- and $H_2$- antagonist activity.

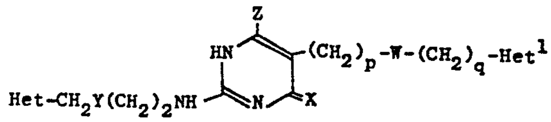
(1)

In Structure (1) Het is a 2- or 4-imidazolyl group optionally substituted by lower alkyl, halogen, trifluoromethyl or hydroxymethyl, a 2-pyridyl group optionally substituted by lower alkyl, lower alkoxy, halogen, amino or hydroxy, a 2-thiazolyl group, a 3-isothiazolyl group optionally substituted by chlorine or bromine, a 3-(1,2,5)-thiadiazolyl group optionally substituted by chlorine or bromine, or a 2-(5-amino-1,3,4-thiadiazolyl) group; Y is sulphur or methylene; Z is hydrogen or lower alkyl; X is oxygen or sulphur; W is methylene, oxygen or sulphur; the sum of p and q is 1 to 4, or, when W is methylene, 0 to 4; and Het[1] is a 5 or 6 membered heterocycle such as pyridine, furan, thiophene, thiazole, oxazole, isothiazole, imidazole, pyrimidine, pyrazine or pyridazine, which heterocycle is optionally substituted by lower alkyl, lower alkoxy, or can have a benzene ring or substituted benzene ring fused to it. One class of compounds described is that in which Het[1] is a 2-furyl, 2-thienyl, 2-pyridyl, 3-pyridyl, 4-pyridyl, 2-thiazolyl, 2-imidazolyl, 2-pyrimidyl, 2-pyrazinyl or 3-pyridazyl group optionally substituted by lower alkyl or lower alkoxy. We have found that a small group of compounds of the general class have improved therapeutic properties, and are less acutely toxic than other members of the general class.

According to the invention there is provided a pyrimidone of Structure (2)

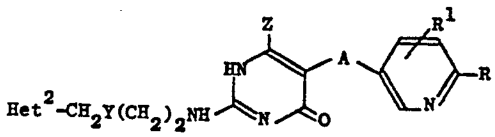
(2)

in which Het[2] is a 4-imidazolyl group optionally substituted by lower alkyl, halogen or trifluoromethyl, a 2-pyridyl group optionally substituted by one or more lower alkyl or lower alkoxy groups or halogen atoms, or a 2-thiazolyl group; Y is sulphur or methylene; Z is hydrogen or lower alkyl; A is $C_1$—$C_5$ alkylene; R is lower alkyl or lower alkoxy; and $R^1$ is hydrogen, lower alkyl or lower alkoxy.

Throughout this specification by the terms 'lower alkyl' and 'lower alkoxy' are meant alkyl and alkoxy groups which can be straight or branched and which contain from 1 to 4 carbon atoms. Particular lower alkyl groups are methyl, ethyl, 1-propyl and 2-propyl. Particular lower alkoxy groups are methoxy, ethoxy, 1-propoxy and 2-propoxy.

The compounds of Structure (2) have particularly good histamine $H_1$- and $H_2$- antagonist activity and are less acutely toxic in mice (when administered intravenously) than the corresponding compounds of Structure (2) in which R is hydrogen. This decrease in acute toxicity is in general is not accompanied by a reduction in histamine $H_1$-antagonist activity or histamine $H_2$-antagonist activity. For example the six specific compounds of Structure (2):—

2-[4-(3-methoxy-2-pyridyl)butylamino]-5-(6-methyl-3-pyridylmethyl)-4-pyrimidone,
2-[4-(3-chloro-2-pyridyl)butylamino]-5-(6-methyl-3-pyridylmethyl)-4-pyrimidone,
2-[2-(5-methyl-4-imidazolylmethylthio)ethylamino]-5-(6-methyl-3-pyridylmethyl)-4-pyrimidone,
2-[2-(5-methyl-4-imidazolylmethylthio)ethylamino]5-(6-methoxy3-pyridylmethyl)-4-pyrimidone,
2-[2-(2-thiazolylmethylthio)ethylamino]-5-(6-methyl-3-pyridylmethyl)-3-pyrimidone, and
2-[2-(3-bromo-2-pyridylmethylthio)ethylamino]-5-(5,6-dimethyl-3-pyridylmethyl)-4-pyrimidone,

when compared with the corresponding 5-(3-pyridylmethyl) analogues which lack the 6-substituent (or 5- and 6-substituents) in the pyridyl group are approximately of equal potency as histamine $H_1$-antagonists and histamine $H_2$-antagonists, but the former have at least three times the $LD_{50}$ value of the latter. ($LD_{50}$ is the minimum dose which kills 50% of mice after bolus intravenous administration of the test compound).

Preferably R is lower alkyl, especially methyl.

When R is lower alkoxy, preferably it is methoxy.

Preferably when $R^1$ is lower alkyl or lower alkoxy it is in the 5-position of the pyridyl group, and especially $R^1$ is the same as R.

Preferably A is straight $\alpha,\omega$-alkylene, particularly methylene.

Preferably Z is hydrogen.

Specific examples of Het[2] are 5-methyl-4-imidazolyl, 5-bromo-4-imidazolyl, 5-trifluoromethyl-4-imidazolyl, 2-pyridyl, 3-methyl-2-pyridyl, 3-methoxy-2-pyridyl, 3-ethoxy-2-pyridyl, 3,4-dimethoxy-2-pyridyl, 3-chloro-2-pyridyl, 3-bromo-2-pyridyl, 3-bromo-4-methyl-2-pyridyl, and 2-thiazolyl.

Preferably Y is methylene and $Het^2$ is a 2-pyridyl group optionally substituted by one or more lower alkyl or lower alkoxy groups or halogen atoms, or a 2-thiazolyl group. Particularly preferably Y is methylene and $Het^2$ is 2-pyridyl, 3-methoxy-2-pyridyl, 3-ethoxy-2-pyridyl, or 3-chloro-2-pyridyl, as these compounds have particularly good histamine $H_1$- and $H_2$-antagonist activity after either oral or intravenous administration.

The compounds of Structure (2) are shown and described as 4-pyrimidone derivatives and these derivatives exist in equilibrium with the corresponding 6-one tautomers. These compounds also exist to a lesser extent as the hydroxy tautomers, and the pyrimidone ring may also exist in the following tautomeric forms:

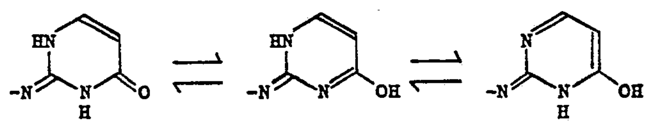

In a process of the invention compounds of Structure (2) are prepared by reacting an amine of Structure (3) where $Het^2$ and Y are as defined above for Structure (2) with a compound of Structure (4),

$$Het^2{-}CH_2Y(CH_2)_2NH_2 \qquad (3)$$

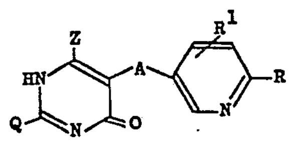

(4)

in which Z, A, R and $R^1$ are as defined above for Structure (2) and Q is nitroamino ($NO_2NH$—), lower alkylthio, benzylthio, chlorine, bromine or other group which can be displaced with a primary amine.

The reaction can be carried out at an elevated temperature in the absence of a solvent, for example at from 80°C to 170°C, preferably from 120°C to 140°C, or in a solvent at an elevated temperature, for example at the reflux temperature of the reaction mixture. The choice of solvent is affected by solubility characteristics of the reactants and the nature of Q. Preferably the solvent is pyridine, a picoline or mixture of picolines, a lower alkanol, preferably ethanol or 1-propanol, an aqueous lower alkanol, 1,2-ethanediol, a ketone, for example acetone or 2-butanone, or a polar aprotic solvent, for example dimethylformamide, dimethylacetamide, dimethylsulphoxide, hexamethylphosphoramide, sulpholane, acetonitrile or nitromethane. Particularly preferably Q is nitroamino and the reaction is carried out in refluxing ethanol, refluxing 1-propanol or refluxing pyridine, or Q is methylthio and the reaction is carried out in refluxing pyridine.

Preferably approximately equimolar amounts of the reactants are used, although an excess, for example a slight excess of from 1.1 to 1.5 molar equivalents or a larger excess of from 1.5 to 4 molar equivalents, of either reactant can be used. If an excess of a reactant is used then preferably an excess of the amine of Structure (3) is used. An excess of either reactant can be present at the start of the reaction or can be added during the course of the reaction.

The starting materials of Structure (4) can be prepared by reacting a β-oxoester of Structure (5)

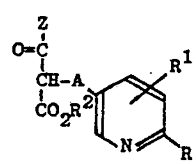

(5)

in which $R^2$ is lower alkyl, with (a) nitroguanidine to give a compound of Structure (4) in which Q is nitroamino, or (b) thiourea followed by an alkylation or benzylation to give a compound of Structure (4) in which Q is lower alkylthio or benzylthio, or (c) guanidine followed by diazotisation in hydrochloric acid in the presence of cuprous chloride and copper or by diazotisation in hydrobromic acid in the presence of cuprous bromide and copper to give a compound of Structure (4) in which Q is chlorine or bromine.

Preferably the reactions of the β-oxoester of Structure (5) with nitroguanidine, thiourea and guanidine are carried out in the presence of a base, for example, an alkali metal lower alkoxide, preferably sodium methoxide or sodium ethoxide, an alkali metal carbonate or hydroxide, preferably potassium carbonate or sodium hydroxide, sodium hydride or a quaternary ammonium hydroxide, for example benzyltrimethylammonium hydroxide. Preferably this reaction is carried out at an elevated temperature, for example the reflux temperature of the solvent mixture. Preferably the solvent is a lower alkanol, for example ethanol, an aqueous lower alkanol, a ketone, for example 2-butanone, or a polar

aprotic solvent, for example dimethylformamide. When Z is hydrogen the β-oxoester of Structure (5) can be used in the form of a hemiacetal of a lower alkanol.

The β-oxoesters of Structure (5) in which Z is hydrogen can generally be prepared by formylating an ester of Structure (6)

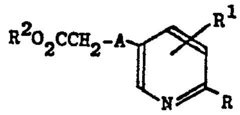

(6)

with a lower alkyl formate, for example ethyl formate, and a strong base, for example sodium hydride in 1,2-dimethoxyethane or tetrahydrofuran, or by using sodium in ether. Preferably in this reaction approximately two molar equivalents of ethyl formate and approximately two molar equivalents of sodium hydride are used. The β-oxoesters of Structure (5) can be used in an unpurified form or in some instances can be isolated and used as crystalline solids. The β-oxoesters of Structure (5) in which Z is lower alkyl can be prepared by alkylating an acylacetate ester.

The amines of Structure (3) in which Y is sulphur can be prepared by reacting cysteamine with a compound of formula $Het^2$-$CH_2L$ where L is a group displaceable with a thiol, for example hydroxy, acyloxy (for example acetoxy, methanesulphonyloxy or toluene-p-sulphonyloxy), lower alkoxy (for example methoxy), chlorine, bromine or triarylphosphonium (for example triphenylphosphonium). Preferably L is hydroxy or methoxy and the reaction is carried out under acidic conditions, for example in hydrochloric or hydrobromic acid.

The amines of Structure (3) in which Y is methylene and $Het^2$ is a 2-pyridyl group with a lower alkoxy group or halogen atom in the 3-position can be prepared by reacting a 2-halo-3-nitropyridine with diethyl 2-(2-cyanoethyl)-malonate. Hydrolysis and decarboxylation of the product followed by reduction with palladium and charcoal gives a 3-amino-3-(3-cyanopropyl)pyridine which can be diazotised in 2M sulphuric acid and alkylated in dimethyl sulphoxide to give a 3-alkoxy-2-(3-cyanopropyl)pyridine. The 3-amino-2-(3-cyanopropyl)pyridine can be reduced with lithium aluminium hydride to give a 4-(3-amino-2-pyridyl)butylamine, which can be diazotised in strong hydrochloric acid in the presence of cuprous chloride to give a 3-chloro amine, or diazotised in strong hydrobromic acid in the presence of cuprous bromide to give a 3-bromo amine, or diazotised in dilute sulphuric acid containing sodium iodide to give a 3-iodo amine. The 3-amino-2-(3-cyanopropyl)pyridines can be diazotised in fluoroboric acid and reduced with lithium aluminium hydride to give a 4-(3-fluoro-2-pyridyl)butylamine.

The amines of Structure (3) in which Y is methylene and $Het^2$ is a 2-thiazolyl group can be prepared by reacting a thioamide of structure $NH_2CS(CH_2)_4Q$, where Q is a protected amino group, with a dialkyl acetal of bromoacetaldehyde and subsequently converting the protected amino group to a free amino group.

The esters of Structure (6) can be prepared by alkylating a dialkyl malonate followed by hydrolysis and decarboxylation, or by condensing an aldehyde with malonic acid and decarboxylating, esterifying and reducing the product.

In this specification by histamine $H_2$-receptors is meant receptors defined by Black et al. (Nature, *236*, 385 (1972)) as those histamine receptors which are not blocked by mepyramine but are blocked by burimamide, and by histamine $H_1$-receptors is meant receptors involved in mepyramine-sensitive histamine responses. Compounds which block histamine $H_2$-receptors are referred to as histamine $H_2$-antagonists and compounds which block histamine $H_1$-receptors are referred to as histamine $H_1$-antagonists.

Blockade of histamine $H_2$-receptors is of value in inhibiting the biological actions of histamine which are not inhibited by histamine $H_1$-antagonists. Histamine $H_2$-antagonists are active, for example, as inhibitors of gastric acid secretion, as antiinflammatory agents and as agents which act on the cardiovascular system, for example as inhibitors of the effects of histamine on blood pressure.

In some physiological conditions the biological actions of histamine are mediated through both histamine $H_1$- and $H_2$-receptors and blockade of both types of receptors is useful. These conditions include inflammation mediated by histamine, for example skin inflammation, and those hypersensitivity responses due to the action of histamine at $H_1$- and $H_2$-receptors, for example allergies.

The activity of the compounds of Structure (2) as histamine $H_2$-antagonists can be demonstrated by the inhibitions of histamine-stimulated secretion of gastric acid from the lumen-perfused stomachs of rats anaesthetised with urethane, at doses of less than 16 micromoles per kilogram intravenously. This procedure is referred to in Ash and Schild, *Brit. J. Pharmac. Chemother., 27,* 427 (1966). Their activity as histamine $H_2$-antagonists can also be demonstrated by their ability to inhibit other actions of histamine which, according to the above mentioned paper of Ash and Schild, are not mediated by histamine $H_1$-receptors. For example, they inhibit the actions of histamine on the isolated guinea pig atrium and isolated rat uterus. They inhibit the basal secretion of gastric acid and also that stimulated by pentagastrin or by food. In a conventional test such as the measurement of blood pressure in the anaesthetised cat, at doses of from 0.5 to 256 micromoles per kilogram intravenously they inhibit the vasodilator action of histamine. The potency of these compounds is illustrated by the effective dose

producing 50% inhibition of gastric acid secretion in the anaesthetised rat and the dose producing 50% inhibition of the histamine-induced tachycardia in the isolated guinea pig atrium (less than $10^{-4}$ Molar).

The activity of the compounds of Structure (2) as histamine $H_1$-antagonists can be demonstrated by the inhibition of histamine-stimulated contractions of the isolated guinea-pig ileum. It is advantageous to administer a single compound having histamine $H_1$- and $H_2$-antagonist activity rather than to administer individual compounds having histamine $H_1$-antagonist activity and histamine $H_2$-antagonist activity as difficulties arising from differing rates of absorption and pharmacokinetic characteristics are avoided.

The invention provides pharmaceutical compositions comprising a pharmaceutical carrier and and a pharmacologically active compound of Structure (2) which can be in the base form or in the form of an addition salt with a pharmaceutically-acceptable acid. Such addition salts include those with hydrochloric, hydrobromic, hydriodic, sulphuric and maleic acids and may conveniently be formed from the corresponding compounds of Structure (2) by standard procedures, for example by treating them with an acid in a lower alkanol or by the use of ion exchange resins to form the required salt either directly from the compound in the base form or from a different addition salt.

The pharmaceutical carrier employed can be a solid or liquid. Examples of solid carriers are lactose, maize starch, potato starch or modified starches, dicalcium phosphate, terra alba, sucrose, cellulose, talc, gelatin, agar, pectin, acacia, magnesium stearate and stearic acid. Examples of liquid carriers are syrup, peanut oil, olive oil, alcohol, propylene glycol, polyethylene glycols and water.

If a solid carrier is used, the composition can be prepared in the form of a tablet, capsule containing powder or pellets, troche or lozenge. The amount of solid carrier in a unit dosage form is generally from about 25 mg to about 300 mg. If a liquid carrier is used, the composition can be in the form of a syrup, emulsion, multiple emulsion, sterile injectable liquid or an aqueous or non-aqueous solution or liquid suspension. Other additives such as preservatives, for example antioxidants or antibacterials, and/or flavouring or colouring agents can also be included. The sterile liquids can be prepared in ampoules, multi-dose vials or unit dose disposable systems. The preparation can also be in a semi-solid form, for example a cream, paste, ointment or gel, or in a liquid or aerosol form for topical application. The pharmaceutical compositions are prepared by conventional techniques involving procedures such as milling, mixing, granulating and compressing, spray drying, freeze drying or dissolving or dispersing the ingredients as appropriate to the desired preparation. The active ingredient is present in the compositions in an effective amount to block histamine $H_2$-receptors. Preferably each dosage unit contains from about 50 mg to about 250 mg of the active ingredient.

The active ingredient is preferably administered from one to six times per day. The daily dosage regimen is preferably from about 150 mg to about 1500 mg. The route of administration can be oral or parenteral.

The preparation of the starting materials is described in the following Preparations and the invention is illustrated by the following Examples: temperatures are in °C.

PREPARATION 1

(i) A mixture of 6-methylpyridine-3-carboxaldehyde (51.57 g), malonic acid (44.30 g.), piperidine (6 ml) and pyridine (300 ml) was stirred at 100° for 3 hours and was allowed to cool. The mixture was evaporated to dryness, water was added to the residue and the solid was filtered off and recrystallised from ethanol-acetic acid to give 3-(6-methyl-3-pyridyl)acrylic acid (41.25 g), m.p. 213.5—215.5°.

(ii) A stirred mixture of 3-(6-methyl-3-pyridyl)acrylic acid (50.70 g), dry ethanol (350 ml) and concentrated sulphuric acid (25 ml) was heated under reflux for 18 hours and ethanol (~250 ml) was removed by evaporation. The residue was poured into ice-aqueous ammonia and the mixture was extracted with ether. The ether extracts were washed with water and evaporated to an oil which crystallised on standing to give ethyl 3-(6-methyl-3-pyridyl)acrylate, m.p. 36—37°C.

(iii) Ethyl 3-(6-methyl-3-pyridyl)acrylate (60.36 g) was hydrogenated in ethanol at 35° and 355 kPa using palladium-on-charcoal catalyst (10%, 1.0 g.). The mixture was filtered and the filtrate was evaporated to give ethyl 3-(6-methyl-3-pyridyl)propionate as an oil.

(iv) Ethyl 3-(6-methyl-3-pyridyl)propionate (57.79 g) and ethyl formate (23.71 g) were added over 2.5 hours to a stirred mixture of sodium wire (6.88 g) and ether (200 ml) cooled in an ice-salt bath. The mixture was stirred for 20 hours and the ether was removed by evaporation. Thiourea (22.76 g) and ethanol (175 ml) were added to the residue and the mixture was heated under reflux for 7 hours and evaporated to dryness. Water (200 ml) was added to the residue and the mixture was adjusted to pH 6 with acetic acid. The solid was filtered off and recrystallised from methanol/acetic acid to give 5-(6-methyl-3-pyridylmethyl)-2-thiouracil (17.24 g), m.p. 240—1°.

(v) Methyl iodide (13.79 g) was added to a stirred solution of 5-(6-methyl-3-pyridylmethyl)-2-thiouracil (22.66 g) and sodium hydroxide (8.0 g) in water (250 ml), and the mixture was heated at 70° for 1 hour and stirred at room temperature overnight. Acetic acid was added until pH 5 and the volume of the mixture was evaporated to a volume of 50 ml. The solid was filtered off and recrystallised from ethanol-acetic acid to give 5-(6-methyl-3-pyridylmethyl)-2-methylthio-4-pyrimidone (10.16 g), m.p-. 197—5°.

PREPARATION 2

(i) A mixture of ethyl 3-(6-methyl-3-pyridyl)propionate (1.31 g) and ethyl formate (7.43 g) was added dropwise to a stirred suspension of sodium hydride (50% dispersion in oil, 4.07 g) in dry 1,2-dimethoxyethane (24 ml) maintained at 0°. The mixture was allowed to warm to room temperature, stirred overnight and poured into ice-water (300 g). The mixture was extracted with ether and the aqueous phase was adjusted to pH 5.4 with hydrochloric acid and the solid which separated was collected to give ethyl 2-formyl-3-(6-methyl-3-pyridyl)propionate (10.5 g, 70%), m.p. 142—4°.

(ii) A solution of ethyl 2-formyl-3-(6-methyl-3-pyridyl)propionate (1.55 g) in methanol (20 ml) was added to a stirred solution of sodium methoxide (from 0.161 g sodium) in methanol (20 ml). Dried nitroguanidine (0.73 g) was added and the mixture was heated under reflux overnight and evaporated to dryness. The residue was dissolved in water (50 ml) and the solution was extracted with chloroform and the aqueous phase was adjusted to pH 5 with acetic acid. The solid which precipitated out was filtered off and recrystallised from methanol-acetic acid to give 2-nitroamino-5-(6-methyl-3-pyridylmethyl)-4-pyrimidone (0.5 g, 27%), m.p. 215—6° (decomp).

PREPARATION 3

(i) A mixture of ethyl formate (111 g) and 2-butanone (108 g) was added dropwise to a stirred mixture of sodium hydride in oil (50% w/w, 72 g) in dry 1,2-dimethoxyethane and the mixture was allowed to stand overnight. Ether (800 ml) was added and the solid (101 g) was filtered off. Cyanoacetamid (69.5 g), piperidine acetate (prepared by adding piperidine to acetic acid (7 ml) and water (18 ml) until the mixture was basic) and water (400 ml) were added to this solid and the mixture was heated under reflux for 2 hours and allowed to cool. The mixture was acidified with acetic acid and the solid which precipitated out was recrystallised from aqueous ethanol to give 3-cyano-5,6-dimethyl-2-hydroxypyridine (43.5 g).

(ii) An intimate mixture of 3-cyano-5,6-dimethyl-2-hydroxypyridine (42 g) and phosphorous pentachloride (81 g) was heated at 140—160° for 2 hours. Phosphoryl chloride was removed by distillation under reduced pressure and ice-water (500 g) was added to the residue. The mixture was adjusted to pH 7 with aqueous sodium hydroxide and extracted with ether. The ether extracts were evaporated to an oil which was crystallised from ether-light petroleum (b.p. 60—80°) to give 2-chloro-3-cyano-5,6-dimethylpyridine (25.3 g), m.p. 83—7°.

(iii) A mixture of 2-chloro-3-cyano-5,6-dimethylpyridine (21.5 g), semicarbazide hydrochloride (24.0 g), sodium acetate (42.3 g), water (225 ml) and methanol (475 ml) was hydrogenated at 344 kPa at 50° using Raney nickel catalyst (5 g). The mixture was added to water (750 ml) and filtered. The solid filtered off was suspended in water (130 ml) and concentrated hydrochloric acid (70 ml) was added and the mixture was heated at 100° for 1 hour; formalin (40% w/w, 120 ml) was added and the mixture was heated at 100° for a further 0.5 hour and allowed to cool. Sodium acetate (95 g) and water (250 ml) were added and the mixture was extracted with ether and the extracts were washed with 5% aqueous potassium carbonate and evaporated to give 2-chloro-5,6-dimethyl-3-pyridinecarboxaldehyde (13.24 g, 60%), m.p. 69—70°.

(iv) A mixture of 2-chloro-5,6-dimethyl-3-pyridinecarboxaldehyde (16.85 g), malonic acid (11.45 g) piperidine (10 ml) and pyridine (100 ml) was heated under reflux for 1 hour and evaporated to an oil. This oil was dissolved in sodium hydroxide solution and was extracted with chloroform, the residual aqueous phase was acidified with hydrochloric acid and extracted with chloroform. The second chloroform extracts were washed with water and evaporated to give 3-(2-chloro-5,6-dimethyl-3-pyridyl)acrylic acid (18.3 g, 87%), m.p. 150—8°. This acid was esterified using ethanol and sulphuric acid to give the ethyl ester, m.p. 85—8°.

(v) The ethyl ester (32.7 g) in ethanol (500 ml) was hydrogenated at 25—30° and 344 pKa using palladium-on-charcoal catalyst (5%, 3 g). The mixture was filtered and the filtrate was evaporated to an oil which was partitioned between chloroform and 2N hydrochloric acid. The aqueous phase was made basic with aqueous sodium hydroxide, extracted with chloroform and the chloroform extracts were evaporated to give ethyl 3-(5,6-dimethyl-3-pyridyl)propionate (21.8 g, 80%) as an oil.

(vi) Reaction of ethyl 3-(5,6-dimethyl-3-pyridyl)propionate with ethyl formate and sodium hydride in dimethoxyethane at room temperature gave ethyl 3-(5,6-dimethyl-3-pyridyl)-2-formylpropionate, m.p. 148—9°.

(vii) Nitroguanidine (6.05 g) was added to a solution of sodium methoxide (prepared from 1.45 g sodium) in dry methanol (65 ml) and the mixture was heated under reflux for 45 minutes. Ethyl 3-(5,6-dimethyl-3-pyridyl)-2-formylpropionate (14.3 g) was added and the mixture was heated under reflux for 40 hours and evaporated to dryness. Water (40 ml) was added to the residue and the mixture was extracted with chloroform. The residual aqueous phase was adjusted to pH 6 with hydrochloric acid and the solid which separated was filtered off and recrystallised from dimethylformamide-ethanol to give 5-(5,6-dimethyl-3-pyridylmethyl)-2-nitroamino-4-pyrimidone, m.p. 212—3°.

PREPARATION 4

(i) A mixture of 2-methoxy-5-cyanopyridine (61.26 g), semi-carbazide hydrochloride (76.4 g), sodium acetate (74.92 g), ethanol (1300 ml) and water (400 ml) was hydrogenated at 344 kPa using

Raney nickel catalyst (1.0 g). The mixture was evaporated to a volume of 500 ml, water (1000 ml) was added and the mixture was allowed to stand at 0° overnight. The mixture was filtered and the solid was washed with water and dissolved in 10% hydrochloric acid (1000 ml). Formaldehyde solution (36% w/v, 450 ml) was added and the mixture was warmed for 15 minutes, allowed to cool and was added to a solution of sodium acetate (298.5 g) in water (900 ml). This mixture was extracted with ether (3 × 500 ml) and the combined extracts were successively washed with aqueous potassium carbonate and water and were dried and evaporated to give 6-methoxypyridine-3-carboxaldehyde (31.5 g 50%), m.p. 48—9°.

(ii) A mixture of 6-methoxypyridine-3-carboxaldehyde (2.34 g), monoethyl malonate (4.51 g), pyridine (12 ml) and piperidine (6 drops) was heated under reflux for 5 hours and was evaporated to an oil. This oil was partitioned between ether and dilute aqueous ammonia. The separated ether layer was washed with water and evaporated to an oil which crystallised on standing to give ethyl 3-(6-methoxy-3-pyridyl)acrylate (2.8 g, 79%), m.p. 49—52°.

(iii) Ethyl 3-(6-methoxy-3-pyridyl)acrylate (32.33 g) in ethanol (160 ml) was hydrogenated at 344 kPa at 40° using palladium-on-charcoal catalyst (5%, 0.2 g). The mixture was filtered and the filtrate was added dropwise over 1.5 hours to a stirred suspension of sodium hydride in oil (50%, 9.38 g)

(iv) A mixture of ethyl 3-(6-methoxy-3-pyridyl)propionate (32.74 g) and ethyl formate (17.22 g) was added dropwise over 1.5 hours to a stirred suspension of sodium hydride in oil (50%, 9.38 g) in 1,2-dimethoxyethane (50 ml) cooled to −2°, allowed to stand overnight at room temperature and then poured on to ice. The mixture was extracted with ether and the aqueous phase was adjusted to pH 5 with 2N sulphuric acid. An oil was precipitated and crystallised on standing to give ethyl 2-formyl-3-(6-methoxy-3-pyridyl)propionate (25.9 g., 70%), m.p. 91.5—94°. A sample recrystallised from aqueous ethanol had m.p. 93—4°.

(v) Nitroguanidine (4.7 g) was added to a solution of sodium methoxide (prepared from 1.15 g sodium) in methanol (50 ml) and the mixture was refluxed for 45 minutes. Ethyl 2-formyl-3-(6-methoxy-3-pyridyl)propionate (10.7 g) was added and the mixture was refluxed for 34 hours and evaporated to a residue. This was dissolved in water and the solution was extracted with chloroform, the residual aqueous solution was adjusted to pH 5 with acetic acid, and the solid which precipitated was filtered off to give 2-nitroaminol-5-(6-methoxy-3-pyridylmethyl)-4-pyrimidone, m.p. 183.5—6°.

## Example 1

A solution of 4-(3-methoxy-2-pyridyl)butylamine (5.48 g) and 2-nitroamino-5-(6-methyl-3-pyridylmethyl)-4-pyrimidone (7.95 g) in pyridine (200 ml) was heated under reflux for 18 hours and evaporated to dryness to give as residue 2-[4-(3-methoxy-2-pyridyl)butylamino]-5-(6-methyl-3-pyridylmethyl)-4-pyrimidone as the crude free base. The residue was extracted with warm propanol (200 ml) and the solution was acidified with ethanolic hydrogen chloride. The solid (10.83 g, 74%) which crystallised out was filtered off and recrystallised from propanol containing 1% 12N aqueous hydrochloric acid to give the trihydrochloride (7.53 g, 51%), m.p. 184—8°.

## Examples 2 to 9

Reaction of equimolar amounts of 5-(6-methyl-3-pyridylmethyl)-2-methylthio-4-pyrimidone and, respectively,

2-(5-methyl-4-imidazolylmethylthio)ethylamine,
4-(5-methyl-4-imidazolyl)butylamine,
4-(2-pyridyl)butylamine,
4-(3-methoxy-2-pyridyl)butylamine,
4-(3-ethoxy-2-pyridyl)butylamine,
4-(3-chloro-2-pyridyl)butylamine,
2-(3-bromo-2-pyridylmethylthio)ethylamine, and
2-(2-thiazolylmethylthio)ethylamine,

by heating at 145—150° and purification of the cold reaction product gave the following compounds.

Example No.

2. 2-[2-(5-methyl-4-imidazolylmethylthio)ethylamino]-5-(6-methyl-3-pyridylmethyl)-4-pyrimidone isolated as the trihyrochloride, m.p. 210—4°.

3. 2-[4-(5-methyll-4-imidazolyl)butylamino]-5-(6-methyl-3-pyridylmethyl)-4-pyrimidone, isolated as the trihydrochloride, m.p. 189—190°.

4. 2-[4-(2-pyridyl)butylamino]-5-(6-methyl-3-pyridylmethyl)-4-pyrimidone, isolated as the free base, m.p. 156—7.5°.

5. 2-[4-(3-methoxy-2-pyridyl)butylamino]-5-(6-methyl-3-pyridylmethyl)-4-pyrimidone, isolated

as a hydrate of the free base, m.p. 67—9°, and converted into the trihydrochloride, m.p. 209—210°.

6. 2-[4-(3-ethoxy-2-pyridyl)butylamino]-5-(6-methyl-3-pyridylmethyl)-4-pyrimidone isolated as the free base, m.p. 104—5°.

7. 2-[4-(3-chloro-2-pyridyl)butylamino]-5-(6-methyl-3-pyridylmethyl)-4-pyrimidone isolated as the free base, m.p. 132—3°.

8. 2-[2-(3-bromo-2-pyridylmethylthio)ethylamino]-5-(6-methyl-3-pyridylmethyl)-4-pyrimidone, isolated as the trihydrochloride, m.p. 193—6°.

9. 2-[2-(2-thiazolylmethylthio)ethylamino]-5-(6-methyl-3-pyridylmethyl)-4-pyrimidone, isolated as the trihydrochloride, m.p. 187—190°.

Example 10

A mixture of 5-(5,6-dimethyl-3-pyridylmethyl)-2-nitroamino-4-pyrimidone and 1.1 equivalents of 2-(5-methyl-4-imidazolylmethylthio)ethylamine was heated at 130° for 5 hours and then heated under reflux in ethanol for 12 hours, evaporated to dryness and the product isolated as 2-[2-(5-methyl-4-imidazolylmethylthio)ethylamino]-5-(5,6-dimethyl-3-pyridylmethyl)-4-pyrimidone monohydrate, m.p. 115° (softening at 98°).

Example 11

A mixture of 5-(5,6-dimethyl-3-pyridylmethyl)-2-nitroamino-4-pyrimidone and 1.2 molar equivalents of 2-(3-bromo-2-pyridylmethylthiol)ethylamine was heated under reflux in ethanol for 48 hours, evaporated to dryness and the product isolated as 2-[2-(3-bromo-2-pyridylmethylthio)ethylamino]-5-(5,6-dimethyl-3-pyridylmethyl)-4-pyrimidone, m.p. 105—7°.

Example 12

Equimolar amounts of 5-(5,6-dimethyl-3-pyridylmethyl)-2-nitroamino-4-pyrimidone and 4-(3-methoxy-2-pyridyl)butylamine were refluxed together in ethanol for 24 hours. An additional 0.1 molar equivalent of the amine was added and the mixture was refluxed for a further 24 hours, evaporated to dryness and purified to give 2-[4-(3-methoxy-2-pyridyl)butylamino]-5-(5,6-dimethyl-3-pyridylmethyl)-4-pyrimidone dihydrate, m.p. 93—94.5°.

Example 13

An equimolar mixture of 2-nitroamino-5-(6-methoxy-3-pyridylmethyl)-4-pyrimidone and 2-(5-methyl-4-imidazolylmethylthio)ethylamine was heated under reflux in ethanol for 18 hours. The solid which crystallised out on cooling was recrystallised from ethanol to give 2-[2-(5-methyl-4-imidazolylmethylthio)ethylamino]-5-(6-methoxy-3-pyridylmethyl)-4-pyrimidone, m.p. 197—8.5°, in 63% yield.

Example 14

When 5,6-dimethoxypyridine-3-carboxaldehyde is converted into 2-nitroamino-5-(5,6-dimethoxy-3-pyridylmethyl)-4-pyrimidone using the procedures of Preparations 1 and 2 and this pyrimidone is reacted with 4-(3-methoxy-2-pyridyl)butylamine, there is obtained 2-[4-(3-methoxy-2-pyridyl)butylamino]-5-(5,6-dimethoxy-3-pyridylmethyl)-4-pyrimidone.

*Preparation of pharmaceutical compositions for oral administration*

A pharmaceutical composition is prepared containing

| | | % w/w |
|---|---|---|
| A | The product of any one of Examples 1 to 14 | 55 |
| | Dibasic calcium phosphate dihydrate | 20 |
| | Approved colouring agent | 0.5 |
| | Polyvinylpyrrolidone | 4.0 |
| B | Microcrystalline cellulose | 8.0 |
| | Maize starch | 8.0 |
| | Sodium starch glycollate | 4.0 |
| | Magnesium stearate | 0.5 |

by mixing together the ingredients A (substituting lactose or microcrystalline cellulose for dibasic calcium phosphate dihydrate if desired), adding a concentrated solution of polyvinylpyrrolidone, and granulating, drying and screening the dried granules; adding the ingredients B to the dried granules and compressing the mixture into tablets, containing an amount of product corresponding to 100 mg, 150 mg or 200 mg of the free base.

*Preparation of pharmaceutical composition for topical administration*

A pharmaceutical composition is prepared containing

| | | % w/w |
|---|---|---|
| A | Stearyl alcohol | 15.0 |
| | Beeswax | 8.0 |
| | Sorbitan monooleate | 1.25 |
| | Polyoxyethylene sorbitan monooleate | 3.75 |
| B | The product of any one of Examples 1 to 14 | 1.0 |
| | Sorbitol solution BP | 7.5 |
| | Citric Acid | 0.2 |
| | Sodium citrate | 0.05 |
| | Methylparaben | 0.18 |
| | Propylparaben | 0.02 |
| | Water | to 100 |

A mixture of the ingredients A is heated to 72°, added with stirring to a mixture of the ingredients B at 70°, and the stirring is continued until a cream is formed.

**Claims**

1. A pyrimidone of Structure (2)

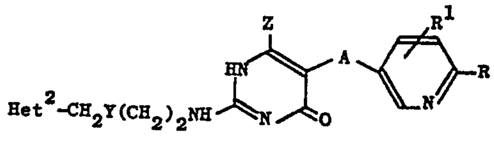

(2)

in which Het is a 4-imidazolyl group optionally substituted by $C_1$—$C_4$ alkyl, halogen or trifluoromethyl, a 2-pyridyl group optionally substituted by one or more $C_1$—$C_4$ alkyl or $C_1$—$C_4$ alkoxy groups or halogen atoms, or a 2-thiazolyl group; Y is sulphur or methylene; Z is hydrogen or $C_1$—$C_4$ alkyl; A is $C_1$—$C_5$ alkylene; R is $C_1$—$C_4$ alkyl or $C_1$—$C_4$ alkoxy; and $R^1$ is hydrogen, $C_1$—$C_4$ alkyl or $C_1$—$C_4$ alkoxy, or a pharmaceutically acceptable acid addition salt thereof.

2. A pyrimidone according to Claim 1, in which R is methyl.

3. A pyrimidone according to Claim 1, in which R is methoxy.

4. A pyrimidone according to any one of Claims 1 to 3, in which $R^1$ is $C_1$—$C_4$ alkyl or $C_1$—$C_4$ alkoxy in the 5-position of the pyridyl group.

5. A pyrimidone according to any one of Claims 1 to 4, in which A is methylene.

6. A pyrimidone according to any one of Claims 1 to 5, in which Z is hydrogen.

7. A pyrimidone according to any one of Claims 1 to 6, in which Y is methylene and $Het^2$ is a 2-pyridyl group optionally substituted by one or more $C_1$—$C_4$ alkyl or $C_1$—$C_4$ alkoxy groups or halogen atoms or a 2-thiazolyl group.

8. A pyrimidone according to any one of Claims 1 to 6, in which Y is methylene and $Het^2$ is 2-pyridyl, 3-methoxy-2-pyridyl, 3-ethoxy-2-pyridyl, or 3-chloro-2-pyridyl.

9. 2-[4-(3-Methoxy-2-pyridyl)butylamino]-5-(6-methyl-3-pyridylmethyl)-4-pyrimidone, or a pharmaceutically acceptable acid addition salt thereof.

10. 2-[4-(3-Methoxy-2-pyridyl)butylamino]-5-(6-methyl-3-pyridylmethyl)-4-pyrimidone trihydrochloride.

11. 2-[4-(3-Chloro-2-pyridyl)butylamino]-5-(6-methyl-3-pyridylmethyl)-4-pyrimidone, or a pharmaceutically acceptable acid addition salt thereof.

12. 2-[2-(5-Methyl-4-imidazolylmethylthio)ethylamino]-5-(6-methyl-3-pyridylmethyl)-4-pyrimidone, or a pharmaceutically acceptable acid addition salt thereof.

13. 2-[2-(5-Methyl-4-imidazolylmethylthio)ethylamino]-5-(6-methoxy-3-pyridylmethyl)-4-pyrimidone, or a pharmaceutically acceptable acid addition salt thereof.

14. 2-[2-(2-Thiazolylmethylthio)ethlamino]-5-(6-methyl-3-pyridylmethyl)-4-pyrimidone, or a pharmaceutically acceptable acid addition salt thereof.

15. 2-[2-(3-Bromo-2-pyridylmethylthio)ethylamino]-5-(5,6-dimethyl-3-pyridylmethyl)-4-pyrimidone, or a pharmaceutically acceptable acid addition salt thereof.

16. A pharmaceutical composition characterised in that it comprises a compound according to any one of Claims 1 to 15 and a pharmaceutically-acceptable diluent or carrier.

17. A process for preparing a compound according to any one of Claims 1 to 15, which comprises reacting a compound of Structure (4),

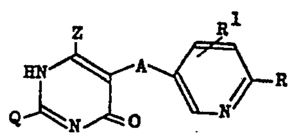

(4)

in which Q is nitroamino, $C_1$—$C_4$ alkylthio, benzylthio, chlorine, bromine or other group which can be displaced with a primary amine, with an amine of structure $Het^2$-$CH_2Y(CH_2)_2NH_2$ and optionally converting the product into a pharmaceutically acceptable acid-addition salt.

**Revendications**

1. Pyrimidone de structure (2)

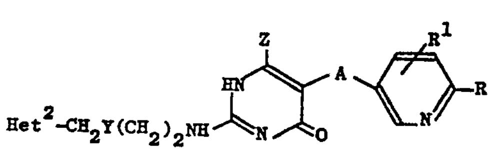

(2)

dans laquelle $Het^2$ représente un groupe 4-imidazolyle éventuellement substitué par un atome d'halogène, un groupe alcoyle en $C_1$—$C_4$ ou trifluorométhyle, un groupe 2-pyridyle éventuellement

substitué par un ou plusieurs groupes alcoyle en $C_1$—$C_4$ ou alcoxy en $C_1$—$C_4$ ou atomes, d'halogène ou un groupe 2-thiazolyle; Y représente un atome de soufre ou un radical méthylène; Z représente un atome d'hydrogène ou un groupe alcoyle en $C_1$—$C_4$; A représente un radical alkylène en $C_1$—$C_5$; R représente un groupe alcoyle en $C_1$—$C_4$ ou alcoxy en $C_1$—$C_4$ et $R^1$ représente un atome d'hydrogène, un groupe alcoyle en $C_1$—$C_4$ ou alcoxy en $C_1$—$C_4$ ainsi que les sels d'addition d'acides pharmaceutiquement acceptables de ces composés.

2. Pyrimidone suivant la revendication 1 dans laquelle R représente un groupe méthyle.

3. Pyrimidone suivant la revendication 1 dans laquelle R représente un groupe méthoxy.

4. Pyrimidone suivant l'une quelconque des revendications 1 à 3, dans laquelle $R^1$ représente un groupe alcoyle en $C_1$—$C_4$ ou alcoxy en $C_1$—$C_4$ substitué en position 5 du groupe pyridyle.

5. Pyrimidone suivant l'une quelconque des revendications 1 à 4 dans laquelle A représente un radical méthylène.

6. Pyrimidone suivant l'une quelconque des revendications 1 à 5, dans laquelle Z représente un atome d'hydrogène.

7. Pyrimidone suivant l'une quelconque des revendications 1 à 6, dans laquelle Y représente un radical méthylène et $Het^2$ représente un groupe 2-pyridyle éventuellement substitué par un ou plusieurs groupes alcoyle en $C_1$—$C_4$ ou alcoxy en $C_1$—$C_4$ ou atomes d'halogène ou un groupe 2-thiazolyle.

8. Pyrimidone suivant l'une quelconque des revendications 1 à 6, dans laquelle Y représente un radical méthylène et $Het^2$ représente un groupe 2-pyridyle, 3-méthoxy-2-pyridyle, 3-éthoxy-2-pyridyle ou 3-chloro-2-pyridyle.

9. 2-[4-3-Méthoxy-2-pyridyl)butylamino]-5-(6-méthyl-3-pyridylméthyl)-4-pyrimidone, ou un sel d'addition d'acide pharmaceutiquement acceptable de ce composé.

10. Trichlorhydrate de 2-[4-(3-méthoxy-2-pyridyl)butylamino]-5-(6-méthyl-3-pyridylméthyl)-4-pyrimidone.

11. 2-[4-(3-Chloro-2-pyridyl)butylamino]-5-(6-méthyl-3-pyridylméthyl)-4-pyrimidone, ou un sel d'addition d'acide pharmaceutiquement acceptable de ce composé.

12. 2-[2-(5-Méthyl-4-imidazolylméthylthio)éthylamino]-5-(6-méthyl-3-pyridylméthyl)-4-pyrimidone, ou un sel d'addition d'acide pharmaceutiquement acceptable de ce composé.

13. 2-[2-(5-Méthyl-4-imidazolylméthylthio)éthylamino]-5-(6-méthoxy-3-pyridylméthyl)-4-pyrimidone, ou un sel d'addition d'acide pharmaceutiquement acceptable de ce composé.

14. 2-[2-(2-Thiazolylméthylthio)éthylamino]-5-(6-méthyl-3-pyridylméthyl)-4-pyrimidone, ou un sel d'addition d'acide pharmaceutiquement acceptable de ce composé.

15. 2-[2-(3-Bromo-2-pyridylméthylthio)éthylamino]5-(5,6-diméthyl-3-pyridylméthyl]-4-pyrimidone, ou un sel d'addition d'acide pharmaceutiquement acceptable de ce composé.

16. Composition pharmaceutique caractérisée en ce qu'elle contient un composé suivant l'une quelconque des revendications 1 à 15 ainsi qu'un diluant ou excipient pharmaceutiquement acceptable.

17. Procédé de préparation d'un composé suivant l'une quelconque des revendications 1 à 15, caractérisée en ce que l'on fait réagir un compsé de structure (4).

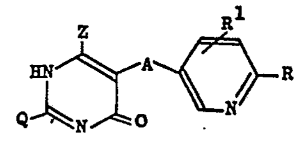

(4)

dans laquelle Q représente un atome de chlore, de brome, un groupe nitroamino, alcoylthio en $C_1$—$C_4$, benzylthio ou un autre groupe qui peut être déplacé par une amine primaire, avec une amine de structure $Het^2$-$CH_2Y(CH_2)_2NH_2$ et qu'on transforme éventuellement le produit en sel d'addition d'acide pharmaceutiquement acceptable.

**Patentansprüche**

1. Eine Pyrimidon der Struktur (2)

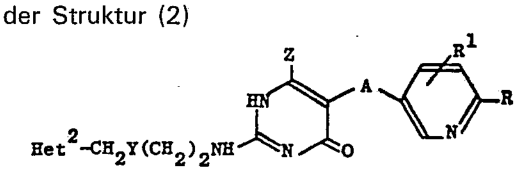

(2)

in der $Het^2$ eine gegebenenfalls mit einem $C_1$—$C_4$-Alkylrest, einem Halogen oder einer Trifluormethylgruppe substituierte 4-Imidazolylgruppe, eine gegebenenfalls mit einem $C_1$—$C_4$-Alkylrest, $C_1$—$C_4$-Alkoxyrest oder Halogenatom einfach oder mehrfach substiuierte 2-Pyridylgruppe, oder eine 2-Thiazolylgruppe ist; Y ein Schwefelatom oder eine Methylengruppe ist; Z ein Wasserstoffatom oder ein $C_1$—$C_4$-Alkylrest ist; A ein $C_1$—$C_5$-Alkylenrest ist; R ein $C_1$—$C_4$-Alkylrest oder ein $C_1$—$C_4$-Alkoxyrest; und $R_1$ ein Wasserstoffatom, ein $C_1$—$C_4$-Alkyl- oder $C_1$—$C_4$-Alkoxyrest ist, oder ein pharmazeutisch verträgliches Säureadditionssalz.

2. Ein Pyrimidon nach Anspruch 1, wobei R ein Methylgruppe ist.

3. Ein Pyrimidon nach Anspruch 1, wobei R eine Methoxygruppe ist.

4. Eine Pyrimidon nach einem der Ansprüche 1 bis 3, wobei $R^1$ ein $C_1$—$C_4$-Alkyl- oder $C_1$—$C_4$-Alkoxyrest in der 5-Stellung der Pyridylgruppe ist.

5. Eine Pyrimidon nach einem der Ansprüche 1 bis 4, wobei A eine Methylgruppe ist.

6. Ein Pyrimidon nach einem der Ansprüche 1 bis 5, wobei Z ein Wasserstoffatom ist.

7. Ein Pyrimidon nach einem der Ansprüche 1 bis 6, wobei Y eine Methylengruppe und $Het^2$ ein 2-Pyridylgruppe ist, die gegebenenfalls mit einem $C_1$—$C_4$-Alkylrest, $C_1$—$C_4$-Alkoxyrest oder Halogenatom ein- oder mehrfach substituiert ist, oder eine 2-Thiazolylgruppe ist.

8. Eine Pyrimidon nach einem der Ansprüche 1 bis 6, wobei Y eine Methylengruppe und $Het^2$ eine 2-Pyridyl-, 3-Methoxy-2-pyridyl-, 3-Äthoxy-2-pyridyl- oder 3-Chlor-2-pyridylgruppe ist.

9. 2-[4-(3-Methoxy-2-pyridyl)-butylamino]-5-(6-methyl-3-pyridylmethyl)-4-pyrimidon oder sein pharmazeutisch verträgliches Säureadditionssalz.

10. 2-[4-(3-Methoxy-2-pyridyl)-butylamino]-5-(6-methyl-3-pyridylmethyl)-4-pyrimidon-trihydrochlorid.

11. 2-[4-(3-Chlor-2-pyridyl)-butylamino]-5-(6-methyl-3-pyridylmethyl)-4-pyrimidon oder sein pharmazeutisch verträgliches Säureadditionssalz.

12. 2-[2-(5-Methyl-4-imidazolylmethylthio)-äthylamino]-5-(6-methyl-3-pyridylmethyl)-4-pyrimidon oder sein pharmazeutisch verträgliches Säureadditionssalz.

13. 2-[2-(5-Methyl-4-imidazolylmethylthio)-äthylamino]-5-(6-methoxyl-3-pyridylmethyl)-4-pyrimidon oder sein pharmazeutisch verträglisches Säureadditionssalz.

14. 2-[2-(2-Thiazolylmethylthio)-äthylamino]-5-(6-methyl-3-pyridylmethyl)-4-pyrimidon oder sein pharmazeutisch veträgliches Säureadditionssalz.

15. 2-[2-(3-Brom-2-pyridylmethylthio)-äthylamino]-5-(5,6-dimethyl-3-pyridylmethyl)-4-pyrimidon oder sein pharmazeutisch verträgliches Säureadditionssalz.

16. Eine pharmazeutische Zusammensetzung, dadurch gekennzeichnet, daß sie eine Verbindung nach einem der Ansprüche 1 bis 15 und ein pharmazeutisch verträgliches Verdünnungsmittel oder einen pharmazeutisch verträglichen Träger enthält.

17. Ein Verfahren zur Herstellung einer Verbindung nach einem der Ansprüche 1 bis 15, gekennzeichnet durch Umsetzung einer Verbindung der Struktur (4)

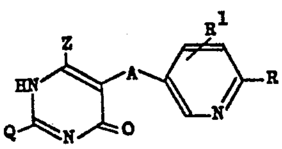

(4)

in der Q eine Nitroaminogruppe, einen $C_1$—$C_4$-Alkylthiorest, eine Benzylthiogruppe, ein Chlor- oder Bromatom oder andere Gruppen bedeutet, die durch ein primäres Amin ersetzt werden können, mit einem Amin der Struktur $Het^2—CH_2Y(CH_2)_2NH_2$, wobei das Produkt gegebenenfalls in das pharmazeutisch verträgliche Säureadditionssalz überführt wird.